# FASCICULE DE BREVET EUROPEEN

(11) **EP 2 531 214 B1**
(45) Date de publication et mention de la délivrance du brevet: **31.08.2016**
(21) Numéro de dépôt: 11704289.5
(22) Date de dépôt: 14.01.2011
(51) Int. Cl.: A61K 39/39, A61K 39/012

(54) **ADJUVANT POUR LA PRÉPARATION DE COMPOSITIONS VACCINALES DESTINÉES À LA PRÉVENTION CONTRE LES COCCIDIOSES**
HILFSMITTEL ZUR ZUBEREITUNG VON IMPFSTOFFZUSAMMENSETZUNGEN ZUR VORBEUGUNG VON KOKZIDIEN
ADJUVANT FOR THE PREPARATION OF VACCINE COMPOSITIONS INTENDED FOR THE PREVENTION OF COCCIDIOSIS

(30) Priorité: 01.02.2010 FR 1050663
(43) Date de publication de la demande: 12.12.2012
(73) Titulaire: Société d'Exploitation de Produits pour les Industries Chimiques SEPPIC, 75007 Paris (FR)
(72) Inventeur: DUPUIS, Laurent, F-51100 Reims (FR); BERTRAND, François, 75014 Paris (FR); DEVILLE, Sébastien, F-75010 Paris (FR)
(74) Mandataire: Grout de Beaufort, François-Xavier
(86) Numéro de dépôt international: PCT/FR2011/050069
(87) Numéro de publication internationale: WO 2011/092413

(56) Documents cités:
- WO-A2-2005/009462
- WO-A2-2006/081826
- Anonymous: "Marcol 52", Exxon Mobil , 2007, pages 1-3, XP002599991, Extrait de l'Internet: URL:http://www.exxonmobil.com/UK-English/S pecialties/PDS/glxxenspcemmarcol_52.pdf [extrait le 2010-09-09]
- Anonymous: "Marcol 82", Exxon Mobil , 2007, pages 1-3, XP002599992, Extrait de l'Internet: URL:http://www.exxonmobil.com/UK-English/S pecialties/PDS/glxxenspcemmarcol_82.pdf [extrait le 2010-09-09]
- DING XICHENG ET AL: "Protective immunity against Eimeria acervulina following in ovo immunization with a recombinant subunit vaccine and cytokine genes", INFECTION AND IMMUNITY, vol. 72, no. 12, décembre 2004 (2004-12), pages 6939-6944, XP002600560, ISSN: 0019-9567
- DUPUIS L ET AL: "SEPPIC vaccine adjuvants for poultry.", ANNALS OF THE NEW YORK ACADEMY OF SCIENCES, vol. 1081, octobre 2006 (2006-10), pages 202-205, XP002599990, ISSN: 0077-8923
- PIRETTI M V ET AL: "INVESTIGATION OF THE HYDRO CARBONS FOUND IN THE TISSUES OF CHICKENS INJECTED WITH INACTIVATED OIL ADJUVANT VACCINE", ZEITSCHRIFT FUER LEBENSMITTEL-UNTERSUCHUNG UND -FORSCHUNG, vol. 175, no. 4, 1982, pages 245-248, XP002599989, ISSN: 0044-3026
- DUNCAN E S STEWART-TULL: "The Use of Adjuvants in Experimental Vaccines. II. Water-in-Oil Emulsions: Freund s Complete and lncomplete Adjuvants", METHODS IN MOLECULAR MEDICINE, vol. 4, 1 janvier 2003 (2003-01-01), pages 141-145, XP001525280, HUMANA PRESS, TOTOWA, NJ, US ISSN: 1543-1894, DOI: 10.1385/0-89603-334-1:141
- AUCOUTURIER JEROME ET AL: "Montanide ISA 720 and 51: a new generation of water in oil emulsions as adjuvants for human vaccines", EXPERT REVIEW OF VACCINES, vol. 1, no. 1, 1 juin 2002 (2002-06-01), pages 111-118, XP002262967, FUTURE DRUGS, LONDON, GB ISSN: 1476-0584, DOI: 10.1586/14760584.1.1.111
- KURODA YOSHIKI ET AL: "Distinctive patterns of autoimmune response induced by different types of mineral oil", TOXICOLOGICAL SCIENCES, vol. 78, no. 2, avril 2004 (2004-04), pages 222-228, XP002599988, ISSN: 1096-6080
- STEWART-TULL DUNCAN E S: "Freund's complete and incomplete adjuvants, preparation, and quality control standards for experimental laboratory animals use", METHODS IN MOLECULAR BIOLOGY, HUMANA PRESS INC, NJ, US, vol. 626, 1 janvier 2010 (2010-01-01), pages 59-72, XP001525277, ISSN: 1064-3745, DOI: DOI:10.1007/978-1-60761-585-9_5
- ISEKI KANAKO ET AL: "Evaluation of a new oil adjuvant for use in peptide-based cancer vaccination.", CANCER SCIENCE OCT 2010 LNKD- DOI:10.1111/J.1349-7006.2010.01653.X PUBMED:20678155, vol. 101, no. 10, octobre 2010 (2010-10), pages 2110-2114, XP002631708, ISSN: 1349-7006

## Description

La présente invention concerne de nouveaux adjuvants pour la préparation de compositions vaccinales destinées à la prévention contre les coccidioses chez les volailles, des compositions vaccinales comprenant lesdits adjuvants, ainsi que l'utilisation d'une huile minérale spécifique pour la fabrication de ce dit adjuvant.
Les coccidioses sont des maladies parasitaires fréquentes chez les volailles.
Une volaille est un oiseau domestique, appartenant généralement aux gallinacés ou aux palmipèdes, élevée pour sa chair ou ses oeufs, soit en basse-cour traditionnelle, soit en élevage industriel.
Par volaille, on entend : le poulet, la dinde, l'oie, le canard, la pintade, le pigeon, la caille, le faisan, l'autruche.

L'agent étiologique (ou porteur de la maladie) est un parasite protozoaire intracellulaire, que l'on nomme coccidie, et appartenant le plus souvent au genre *Eimeria.*

Il existe plusieurs types de coccidies pour chaque espèce aviaire :
Coccidies du poulet : *E. acervulina, E. necatrix, E. maxima, E. brunetti, E. tenella, E. mitis, E. praecox.*
Coccidies de la dinde : *E. meleagrimitis, E. adenoeides, E. dispersa, E. gallopavonis.*
Coccidies de l'oie : *E. truncata* (elle peut aussi toucher le canard de Barbarie et le cygne), *E. anseris.*
Coccidies du canard : *Tyzzeria perniciosa, E. mulardi.* La maladie concerne surtout le canard mulard.
Coccidies de la pintade : *E. numidia, E. grenieri* (la plus fréquente mais au pouvoir pathogène inférieur).
Coccidies du pigeon : *E. labbeana*
La prévention de la coccidiose repose sur les solutions suivantes :

### a) une méthode de chimio-prévention:

Cette méthode met en oeuvre des composés chimiques de type anti-biotiques et possède comme inconvénients :
- de générer des risques de développement d'allergies chez les animaux traités avec ces antibiotiques, notamment en raison de la présence de « résidus » ou de sous-produits non désirés dans ces antibiotiques ;
- de générer des risques de développement de résistance face aux antibiotiques chez les espèces traitées ;
- d'être régie par une réglementation contraignante ;
- de dénaturer l'image de la viande des volailles commercialisée, avec le risque de ne plus bénéficier des labels de qualité revendiquant un élevage dans des conditions « naturelles ».

### b) une méthode de vaccination impliquant des vaccins « vivants » :

Les vaccins issus d'agents vivants atténués sont préparés par la multiplication des agents infectieux en laboratoire, jusqu'à ce qu'ils perdent naturellement ou artificiellement, par mutation, leur caractère pathogène.

Les souches obtenues sont alors incapables de développer entièrement la maladie qu'elles généraient auparavant.

Ces souches conservent cependant leurs antigènes, et leur capacité à induire des réponses immunitaires.
Ce mode de vaccination permet, après administration aux animaux, de coloniser les voies digestives et d'éviter l'implantation de souches possédant des facteurs de virulence.
Ce mode de vaccination présente comme inconvénients :
- de possibles retours à la virulence ;
- de devoir réaliser des cultures à grande échelle de bactéries potentiellement pathogènes ;
- les animaux vaccinés selon ce mode peuvent excréter, constituant ainsi une source potentiellement pathogène dans l'environnement et donc des potentiels de dissémination de ces pathogènes.

Ce dernier inconvénient constitue donc une raison pour laquelle il n'est pas privilégié dans le cas des élevages en plein air des volailles pour vacciner contre la coccidiose qui est une maladie parasitaire et donc facilement transmissible par dissémination.

Les vaccins anticoccidiens sont des médicaments vétérinaires. De ce fait, ils sont soumis à la législation s'appliquant aux médicaments vétérinaires, dépendant elle-même de la législation sur les médicaments.

Trois vaccins anticoccidiens sont autorisés en France : ce sont des vaccins vivants constitués de souches "précoces", atténuées mais immunogènes et protectrices vis-à-vis des espèces présentes sur le terrain.

Ces trois vaccins ne sont utilisables que pour l'espèce poule *(Gallus gallus*) car ils contiennent seulement des espèces susceptibles de parasiter cette espèce d'oiseau, il n'existe pas d'immunité croisée vis-à-vis des différentes espèces de coccidies.

### c) les méthodes de vaccination conventionnelles avec un antigène non vivant ou « inactivé » :

L'efficacité des vaccins existants ne donne pas satisfaction, car le niveau de la composante cellulaire de la réponse immunitaire n'est pas assez élevé pour induire des protections efficaces dans le cas spécifique de maladies parasitaires comme la coccidiose en particulier.

Beaucoup de ADNc codant pour des antigènes *d'Eimeria* ont été décrits, et des essais d'immunisation sont en cours avec certains d'entre eux. Le développement de la résistance dépend aussi du fond génétique et du mode d'administration (et de l'adjuvant). Certains antigènes ont montré une protection partielle. La recherche vise des antigènes communs à plusieurs espèces de coccidies : par exemple, l'antigène GX3262 réactif avec un anticorps monoclonal qui reconnaît un antigène de sporozoïte commun aux sept espèces de coccidies de poulet, induit une protection partielle.

Il y a donc un besoin d'améliorer ces compositions vaccinales inactivées.

Le développement de vaccins inactivés ou contenant des antigènes purifiés est de plus en plus important car il permet d'éviter au maximum les effets secondaires indésirables chez les sujets vaccinés pendant et après la vaccination. Cependant l'amélioration de la qualité des antigènes se fait au détriment de leur immunogénicité. C'est pour cette raison qu'ils sont associés à des adjuvants permettant d'augmenter la réponse immunitaire chez les sujets vaccinés.

Ces adjuvants sont de natures diverses. Ils peuvent par exemple consister en des liposomes, des émulsions comprenant au moins une phase huile et au moins une phase aqueuse, du type adjuvants dits de Freund ou, de manière plus courante en des sels minéraux insolubles dans l'eau. Parmi les sels minéraux utilisés comme adjuvants de compositions vaccinales, on peut citer par exemple l'hydroxyde d'aluminium, le nitrate de cérium, le sulfate de zinc, l'hydroxyde de fer colloïdal ou le chlorure de calcium. L'hydroxyde d'aluminium est l'adjuvant le plus couramment utilisé. Ces sels minéraux utilisés comme adjuvants de compositions vaccinales sont décrits notamment dans l'article de Rajesh K. Gupta et al "Adjuvants, balance between toxicity and adjuvanticity", Vaccine, vol. 11, Issue 3, 1993, pages 993-306.

Les adjuvants mentionnés ci-dessus présentent comme inconvénient une efficacité faible. En outre, il est connu que l'hydroxyde d'aluminium n'induit efficacement qu'une immunité humorale, et non une immunité cellulaire. Par ailleurs, ils peuvent induire une certaine toxicité vis-à-vis des sujets traités. Plus particulièrement, lorsque ces compositions vaccinales sont injectées aux sujets à vacciner, on peut observer la formation de lésions et d'autres réactions locales telles que des granulomes au niveau du point d'injection.

D'autres adjuvants, constitués de sels de métaux divalents ou trivalents ou encore de composés sympathomimétiques sont décrits respectivement dans les demandes internationales de brevet publiées sous les numéros WO 96/32964, WO 98/17311 et WO 98/15288.

Les émulsions sont des mélanges stables constitués par un système de deux liquides non miscibles dont l'un est divisé en gouttelettes dans l'autre. Ainsi, les émulsions comprennent des huiles, une phase aqueuse et des agents tensioactifs qui ont pour fonction de disperser l'une des deux phases dans l'autre et d'obtenir un mélange macroscopiquement homogène et stable. Elles sont souvent utilisées comme adjuvants de l'immunité dans les formulations vaccinales (voir par exemple l'article de J. Aucouturier et al. "Montanide ISA 720 and 51: a new generation of water in oil emulsions as adjuvants for human vaccines", Expert Review of Vaccines, vol. 1, no. 1, 1 juin 2002, pages 111-118).
L'utilisation d'huiles minérales comme adjuvant de vaccin est connues depuis de nombreuses années (Freund 1956, Herbert 1967). Les huiles minérales utilisées comme adjuvants de vaccins sont des hydrocarbures liquides, qui sont obtenues par distillation du pétrole et par la mise en oeuvre d'étapes de traitement subséquentes comme par exemple les étapes de désulfurisation, de désasphaltage, d'extraction des composés aromatiques, d'extraction des cires, et autres étapes de traitement de finition. Les études de l'influence de la qualité des huiles sur la réponse immunitaire n'est venue que progressivement et de manière incomplète. En effet, la composition des huiles minérales est très influencée par l'origine géographique des bruts utilisés ainsi que du procédé (chimie et équipement) mis en oeuvre pour leur préparation. Les études effectuées sur les différentes fractions étaient destinées à évaluer le caractère toxique pour les travailleurs utilisateurs mais pas pour des utilisations en santé animale ou humaine). Les optimisations de composition de ces huiles minérales, basées sur des caractérisations macroscopiques (viscosité, point de fusion) ont été effectuées avec des grades d'huiles minérales industrielles existantes, ou de produits chimiques purs de laboratoires ne correspondant pas à des définitions ni à la caractérisation de grades de matières premières acceptables. Ainsi, les études même poussées, effectuées sur des grades industriels d'huiles minérales définies par des paramètres ne reflétant par leurs propriétés biologiques entraînaient et entraînent encore des variations de comportements lors de l'utilisation pour des vaccins en fonction de l'origine d'un lot d'huile minérale. Ces lots, bien que répondant à leur cahier des charges et spécifications analytiques macroscopiques et portant le même nom commercial, ont des compositions suffisamment différentes pour ne pas donner les mêmes propriétés aux vaccins. Par ailleurs, la viscosité des émulsions obtenues à partir des huiles minérales, sans optimisation galénique, induisait un biais dans l'interprétation des résultats.

L'influence de la répartition entre chaînes linéaires et cyclisée a été évaluée pour rechercher une éventuelle toxicité lors de l'utilisation des huiles minérales en injectable suspectant le rôle tératogène des formes cyclisées. Les critères de toxicités ont ensuite été identifiés comme provenant des formes insaturées, éliminées parfaitement dans les procédés actuels par hydrogénation poussée.

Aucune étude n'a été effectuée dans le but d'évaluer l'influence du ratio de constituants de type linéaires ou cyclisés sur la qualité de la réponse immunitaire. Les huiles minérales ont été utilisées depuis plusieurs décennies comme adjuvants et pour la préparation d'adjuvants destinés à augmenter l'efficacité de la réponse immunitaire des vaccins. En effet, des antigènes préalablement émulsionnés dans des émulsions de type eau dans huile minérale (E/H) puis administrés par voie parentérale déclenchent des réponses immunitaires (et par conséquence des protections contre les pathogènes) beaucoup plus intenses et durables.
La nature de l'huile minérale utilisée pour la préparation d'adjuvant a une influence importante sur l'intensité de la réponse immunologique et sur les réactions secondaires liées à l'injection du vaccin (réactions locales et générales).

Il y a deux types de réponses immunitaires :

### - La réponse immunitaire humorale :

c'est la réaction qui se produit lorsque des lymphocytes B possédant des récepteurs spécifiques sont stimulés par un antigène et se différencient en clone de plasmocytes qui commencent à sécréter des anticorps. Ceux-ci sont efficaces contre les agents pathogènes circulant dans le sang et la lymphe. De plus, l'activation sélective des lymphocytes B dote l'organisme de cellules mémoires à durée de vie prolongée qui interviennent dans la réponse immunitaire secondaire, et

### - La réponse immunitaire cellulaire

La réaction immunitaire humorale aide le réseau de défense à reconnaître et détruire les agents pathogènes libres, mais c'est la réaction à médiation cellulaire qui combat les agents pathogènes déjà introduits dans les cellules. Les principaux acteurs de l'immunité à médiation cellulaire sont les lymphocytes T.
Les lymphocytes T ne réagissent qu'aux déterminants antigéniques exposés à la surface des cellules de l'organisme. Les lymphocytes T reconnaissent ces déterminants grâce à leurs récepteurs, des protéines surfaciques enchâssées dans leur membrane plasmique. Le récepteur du lymphocyte T reconnaît l'Antigène combiné avec une des glycoprotéines du CMH de l'organisme. Outre leur rôle dans la réponse humorale, les Lymphocytes T auxiliaires peuvent également activer d'autres types de lymphocytes T pour déclencher les réactions à médiation cellulaire contre des Antigènes. Les Lymphocytes T cytotoxiques sont les seules cellules à tuer d'autres cellules (celles infectées par des agents pathogènes intra cellulaires).

Les adjuvants huileux sont très connus pour augmenter la composante humorale de la réponse immunologique (mesurée en général par des dosages d'immunoglobulines du groupe 1 ou IGG1) alors que des réponses de type cellulaires (mesurées indirectement par les IGG2) sont très difficiles à obtenir.

Il est connu que les émulsions E/H, donnant les plus fortes réponses immunitaires, induisent aussi une composante de type cellulaire mais dans des proportions moindres par exemples que certaines molécules solubles (type saponines ou tensioactifs cationiques) réputées pour orienter la réponse vers des mécanismes cellulaires.

Les émulsions à phase continue aqueuse utilisant les huiles minérales classiquement sur le marché, sont de très mauvais promoteurs de la réponse cellulaire.

En effet, le type de réponse engendrée par les vaccins est influencé par la structure de l'antigène, mais également par la façon dont celui ci est présenté au système immunitaire via les adjuvants. Les adjuvants ont différents effets sur la réponse immunitaire, car ils peuvent induire une réponse immunitaire sur une durée plus ou moins longue, avec un caractère prédominant de type cellulaire ou de type humoral.

L'immunité à médiation cellulaire est importante, voire parfois essentielle pour la protection contre les bactéries intracellulaires, les virus et la plupart des parasites. Elle intervient dans la plupart des mécanismes de protection vaccinale en complément de la réponse humorale.

On pourra par exemple classer la capacité d'un adjuvant à induire une réponse immunologique de type cellulaire pour la composition vaccinale le comprenant en comparant les rapports IGG1/IGG2 mesurés suite à l'administration par voie parentérale de la composition vaccinale le comprenant aux sujets concernés, à des périodes différentes suivant le jour de vaccination desdits sujets. Plus ce rapport sera élevé et plus l'adjuvant aura orienté la réponse immunitaire de la composition vaccinale vers une composante humorale. Une valeur du rapport IGG1/IGG2 proche de l'unité sera considérée comme le signe d'une réponse immunitaire de la composition vaccinale équilibrée entre ses deux composantes. Une valeur du rapport IGG1/IGG2 inférieure à l'unité sera caractéristique d'une réponse immunitaire de la composition vaccinale à prédominance cellulaire.

Un but de la présente invention est de disposer d'un adjuvant pour la préparation de compositions vaccinales, destiné à améliorer la composante cellulaire de la réponse immunitaire, tout en maintenant le bon niveau de la composante humorale. Il y a donc un besoin d'obtenir des adjuvants de vaccins ayant une réponse immunitaire équilibrée voire à prédominance cellulaire. En outre les vaccins préparés avec l'adjuvant objet de la présente invention devront présenter une bonne innocuité.

A cette fin, la présente invention, a pour objet un adjuvant de vaccin tel que défini à la revendication 1 et son utilisation pour la fabrication d'une composition vaccinale destinée à la prévention contre la coccidiose telle que définie aux revendications 2 et 3.

Selon un autre aspect, l'invention a pour objet un vaccin tel que défini aux revendications 4 à 8.

Selon un autre aspect, l'invention a pour objet l'utilisation d'une huile minérale pour la fabrication d'un vaccin tel que défini ci-dessus, caractérisée en ce que la dite huile comprend pour 100% de sa masse :
- de 0,05% à 10% de chaînes hydrocarbonées ayant moins de 16 atomes de carbone ;
- de 0,05% à 5% de chaînes hydrocarbonées ayant plus de 28 atomes de carbone ;
et possède un rapport P/N, correspondant au rapport de la quantité massique de chaînes carbonées de type paraffinique sur la quantité massique de chaînes carbonées de type naphténique, compris entre 2,5 et 3, de préférence entre 2,8 et 2,9.

La composition selon l'invention peut également avantageusement comporter un ou plusieurs agents tensioactifs émulgateurs. Ce dernier présente un caractère lipophile ou hydrophile caractérisé par une valeur HLB (hydrophile-lipophile-balance) comprise entre 1 et 19.

Un tel tensioactif peut consister en
- un alkylpolyglycoside ou un mélange d'alkylpolyglycosides de formule Rₐ-(O)-Zn où Rₐ représente un radical aliphatique saturé, linéaire ou ramifié, comprenant de 4 à 24 atomes de carbone, Z est le reste d'un sucre, de préférence le glucose et n est compris entre 1 et 5 de préférence entre 1,1 et 2,
- les saponines,
- les esters de sorbitan, comme par exemple l'oléate de sorbitan, le stéarate de sorbitan, le palmitate de sorbitan, le laurate de sorbitan ;
- les esters mannitan, comme par exemple l'oléate de mannitan, le stéarate de mannitan, le palmitate de mannitan, le laurate de mannitan ;
- les esters de sorbitan polyoxyéthylés entre 5 moles et 20 moles d'oxyde d'éthylène, comme par exemple l'oléate de sorbitan éthoxylé à 20 moles d'oxyde d'éthylène ;
- les esters de mannitan polyoxyéthylés entre 5 moles et 20 moles d'oxyde d'éthylène, comme par exemple l'oléate de mannitan éthoxylé à 20 moles d'oxyde d'éthylène ;
- les lécithines ;
- les alcanols polyoxyéthylés comme par exemple ceux commercialisés sous l'appellation BRIJ, et plus particulièrement le BRIJ 21 et le BRIJ 221 par la société UNIQEMA ;
- les polymères tensioactifs comprenant des blocs polyoxyéthylènes et polyoxypropylènes, tels que ceux commercialisés sous la désignation PLURONICS par la société BASF ;
- les polyhydroxystéarates de polyglycol ou de polyglycérol comme par exemple les produits dénommés HYPERMER ™ B246, ARLACEL™P135 commercialisés par la société UNIQEMA.

Par antigène on entend toute molécule ayant des propriétés immunogéniques pouvant induire une réponse immunitaire spécifique. Ces antigènes peuvent être des pathogènes inactivés, comme par exemple des bactéries inactivées, des molécules extraites des pathogènes, des molécules produites par recombinaison génétique de microorganismes.

Les huiles minérales sont obtenues à partir de la distillation puis de l'hydrogénation poussée de produits d'origine pétrolière. Elles sont constituées par des molécules chimiques de la famille des alcanes saturés. Pour ce type de molécules, le nombre d'atomes de carbone (n) est en général supérieur à 9 et inférieur à 30, conférant alors un aspect liquide à 25°C. On parle aussi de paraffines liquides. Les paraffines solides sont constituées par des molécules chimiques de la famille des alcanes pour lesquelles n est supérieur à 30, pouvant aller jusqu'à 50 et confèrent alors un aspect solides à 25°C. Les paraffines liquides, ou huiles minérales, sont des liquides transparents et inodores, dont la viscosité dynamique peut varier de 2 milliPascal.secondes (mPa.s) à 1 Pascal.seconde. La viscosité dynamique des huiles minérales est directement liée à la longueur moyenne des chaînes carbonées des alcanes composant lesdites huiles minérales. Les alcanes à chaînes hydrocarbonées les plus longues sont responsables des viscosités dynamiques les plus élevées. Les molécules concernées peuvent se présenter sous forme de chaînes linéaires hydrocarbonées ou linéaires branchées (regroupées sous la dénomination « paraffinique » (P) ou cyclisées (naphténiques N)) mais toujours saturées (sans doubles liaisons qui sont éliminées lors du traitement par l'hydrogénation). Les viscosités, points de fusion, et points d'ébullition sont directement liés au nombre de carbone que présente la chaîne hydrocarbonée. Une huile blanche minérale pourra donc être caractérisée par son intervalle de distillation, sa viscosité dynamique, son nombre d'atomes de carbone moyen, mais aussi par sa fraction P/N en molécules linéaires et branchées sur la quantité des molécules cyclisées (déterminée selon la méthode DIN 51378). A noter que le critère de viscosité dynamique peut être modifié artificiellement par mélange de deux coupes différentes.

### EXEMPLES

### I) PREMIERE PARTIE

Les exemples suivants permettent de mettre en évidence les propriétés des compositions adjuvantes selon la présente invention.

Dans ce travail, une étude des propriétés adjuvantes de différentes huiles caractérisées finement à permis de mettre en évidence des compositions originales nouvelles caractérisées par des limites de compositions établies par chromatographie.

L'étude effectuée sur des compositions vaccinales expérimentales dans un premier temps avec des formules se présentant sous la forme d'émulsions Eau-dans-huile (E/H) à permis d'établir les conditions optimales d'obtention de réponses immunitaires équilibrées entre ses deux composantes et d'une réponse immunitaire à composante cellulaire prédominante pour des formules bien tolérées sur des souris. La suite des essais expérimentaux, réalisés sur d'autres types de formules et différents animaux a permis de mettre en évidence que les compositions adjuvantes objets de la présente invention assurent les meilleures réponses immunologiques tant quantitatives que qualitatives.

Dans les exemples qui suivent, les huiles minérales sélectionnées sont caractérisées par leur:
- Fraction courte (FC) exprimé en pourcent, correspondant à la proportion d'entités ayant un nombre d'atomes de carbone C < 16.
- Fraction lourde (FL) exprimé en pourcent, correspondant à la proportion d'entités ayant un nombre d'atomes de carbone C > 28.
- Répartition moléculaire (RM) (exprimée en nombre d'atomes de carbone C) calculée comme le barycentre de la courbe comprise entre C16 et C28 en attribuant le coefficient surfacique des fractions C16 à C20 assimilées à 18 atomes de carbone, 20 atomes de carbone à 24 atomes de carbone assimilées à 22 atomes de carbone et 24 atomes de carbone à C28 assimilées à 26 C.
- le rapport P/N (obtenu selon la méthode normalisée DIN 51378).

Les différentes huiles testées sont listées dans le tableau 1.

**Tableau 1 : panel d'huiles synthétisées et caractérisées pour essais biologiques.**

| **Ref Huile** | **FC** | **RM** | **FL** | **P/N** |
|---|---|---|---|---|
| | (**<C16**) | | **(>C28)** | |
| 1 | 13.7 | 20.6 | 3.3 | 1.78 |
| 2 | 4.9 | 21.5 | 7.1 | 1.86 |
| 3 | 4.2 | 20.5 | 1.5 | 1.86 |
| 4 | 4.4 | 21.6 | 4.7 | 1.86 |
| 5 | 47.7 | 18.4 | 0.5 | 1.86 |
| 6 | 0.3 | 22.2 | 9.6 | 2.03 |
| 7 | 30 | 18.3 | 0.6 | 2.19 |
| 8 | 16 | 21.8 | 10 | 2.13 |
| 9 | 2 | 19.3 | 2.2 | 2.85 |
| 10 | 48.2 | 18.4 | 0.6 | 1.86 |
| 11 | 8.6 | 20.7 | 2.4 | 1.94 |
| 12 | 20.8 | 21.9 | 8.7 | 1.86 |

### ETUDE SOUS LA FORME D'EMULSION EAU DANS HUILE E/H :

La formulation est conduite dans un modèle type adjuvant incomplet de Freund (IFA) utilisé à 50% dans l'émulsion vaccinale qui par la simplicité de sa formule (mannide monooléate 15% + huile minérale 85%) permet une comparaison pertinente.

Les propriétés biologiques des vaccins ainsi formulés (50% IFA + 50% de solution isotonique d'albumine d'oeuf à 10 µg / dose ; 1 dose = 100 µl d'émulsion) ont alors été comparées sur modèle souris pour les paramètres d'innocuité (tableau 2), et d'efficacité pour la réponse humorale ou cellulaire.

L'innocuité est évaluée par observation du site d'injection 7 jours après vaccination puis notation des réactions locales (RL) sur une échelle allant de 0 à 4. Les valeurs de 0 à 2 sont acceptables (il s'agit au maximum de dépôt huileux sans alopécie, pas d'induration palpable ni site d'injection visible) ; les valeurs supérieures à 2 sont considérées comme non souhaitées et inacceptables (il s'agit de nécroses, alopécies, induration palpables). Les résultats sont groupés dans le tableau 2 ; les huiles minérales testées dans la préparation de la formulation E/H ont été classées en fonction de leur FC. Il apparaît très clairement que les formulations E/H comprenant des huiles minérales ayant un FC < à 13,7 sont appropriées pour la préparation de compositions vaccinales utilisables en injectable.

**Tableau 2 : évaluation de l'innocuité des différentes huiles.**

| **Ref Huile** | **FC (<C16)** | **RM** | **FL (>C28)** | **P/N** | **RL** |
|---|---|---|---|---|---|
| 1 | 13.7 | 20.6 | 3.3 | 1.78 | 2.5 |
| 2 | 4.9 | 21.5 | 7.1 | 1.86 | 0.5 |
| 3 | 4.2 | 20.5 | 1.5 | 1.86 | 0 |
| 4 | 4.4 | 21.6 | 4.7 | 1.86 | 0.5 |
| 5 | 47.7 | 18.4 | 0.5 | 1.86 | 4 |
| 6 | 0.3 | 22.2 | 9.6 | 2.03 | 0 |
| 7 | 30 | 18.3 | 0.6 | 2.19 | 3 |
| 8 | 16 | 21.8 | 10 | 2.13 | 2.5 |
| 9 | 2 | 19.3 | 2.2 | 2.85 | 0 |
| 10 | 48.2 | 18.4 | 0.6 | 1.86 | 4 |
| 11 | 8.6 | 20.7 | 2.4 | 1.94 | 0.5 |
| 12 | 20.8 | 21.9 | 8.7 | 1.86 | 3 |

Les formules retenues ont ensuite été testées pour leur efficacité immunologique dans le modèle souris. Les formules donnant des réactions inacceptables ont été éliminées. Les tableaux 3 et 4 montrent les titres anticorps obtenus, comparés à une référence d'hydroxyde d'aluminium (adjuvant solide minéral utilisé en médecine vétérinaire et humaine). Les titres sont donnés à 14/28/42/56/90 jours après la date des premières injections, pour la réponse humorale (IGG1) et cellulaire (IGG2). Une seconde injection des formules est réalisée à 28 jours, après l'enregistrement des titres anticorps. Les résultats pour la réponse à long terme (90 jours) indiquent une corrélation forte entre des faibles valeurs de fraction lourde et l'intensité de la réponse humorale à 90 jours. La réponse est significativement diminuée lorsque cette fraction est supérieure à 4.7% (7.1% et 9.6%).

Ces résultats démontrent qu'une huile minérale pour adjuvant de vaccin sous forme d'émulsion E/H contenant moins de 10% de FC, moins de 5% de FL et un ratio P/N supérieur ou égal à 1,9 induit une bonne réponse de type cellulaire.

**Tableau 3 : Réponse anticorps de type IgG1 contre OVA dans la souris pour différentes huiles après injection sous forme émulsion E/H en fonction du temps.**

| **HUILE REF** | **FC (<C16)** | **RM** | **FL (>C28)** | **P/N** | **J14** | **J28** | **J42** | **J56** | **J90** |
|---|---|---|---|---|---|---|---|---|---|
| 2 | 4.9 | 21.5 | 7.1 | 1.86 | 9600 | 16 000 | 128 000 | 128 000 | 128 000 |
| 3 | 4.2 | 20.5 | 1.5 | 1.86 | 9600 | 16 000 | 128 000 | 256 000 | 256 000 |
| 4 | 4.4 | 21.6 | 4.7 | 1.86 | 9600 | 16 000 | 128 000 | 256 000 | 256 000 |
| 6 | 0.3 | 22.2 | 9.6 | 2.03 | 9600 | 16 000 | 64 000 | 64 000 | 64 000 |
| 9 | 2 | 19.3 | 2.2 | 2.85 | 9600 | 16 000 | 128 000 | 256 000 | 256 000 |
| 11 | 8.6 | 20.7 | 2.4 | 1.94 | 9600 | 16 000 | 128 000 | 256 000 | 256 000 |
| AlOH | | | | | 16 000 | 4 800 | 4 800 | 2400 | 2400 |

**Tableau 4 : Réponse anticorps de type IgG2 contre OVA dans la souris pour différentes huiles après injection sous forme émulsion E/H.**

| **Ref Huile** | **FC** (**<C16**) | **RM** | **FL (>C28)** | **P/N** | **J14** | **J28** | **J42** | **J56** | **J90** |
|---|---|---|---|---|---|---|---|---|---|
| 2 | 4.9 | 21.5 | 7.1 | 1.86 | 9600 | 16 000 | 48 000 | 48 000 | 48 000 |
| 3 | 4.2 | 20.5 | 1.5 | 1.86 | 9600 | 16 000 | 48 000 | 48 000 | 48 000 |
| 4 | 4.4 | 21.6 | 4.7 | 1.86 | 9600 | 16 000 | 48 000 | 48 000 | 48 000 |
| 6 | 0.3 | 22.2 | 9.6 | 2.03 | 9600 | 16 000 | 128 000 | 256 000 | 256 000 |
| 9 | 2 | 19.3 | 2.2 | 2.85 | 9600 | 16 000 | 128 000 | 256 000 | 256 000 |
| 11 | 8.6 | 20.7 | 2.4 | 1.94 | 9600 | 16 000 | 128 000 | 256 000 | 256 000 |
| AlOH | | | | | 16 000 | 4 800 | 4 800 | 2400 | 2400 |

### ETUDE SOUS LA FORME D'EMULSION A PHASE CONTINUE AQUEUSE :

Deux types galéniques représentant les émulsions à phase continue aqueuse (multiple E/H/E et microémulsion H/E) ont été testés.

### Microémulsion Huile dans eau :

La formule adjuvante comprend pour 100% de sa masse 40% massique d'un tensioactif hydrophile (le polysorbate 80) et 60% massique d'huile minérale à tester ; la phase huileuse ainsi formulée est émulsionnée à température ambiante par un homogénéisateur à haute pression en présence d'une phase aqueuse comprenant l'antigène (OVA) ; la phase huileuse précédemment préparée est utilisée à 10% massique dans la composition vaccinale finale (OVA 10 µg/dose, 100 µl par injection); un témoin sur hydroxyde d'aluminium est inclus dans l'essai. Les résultats sont présentés dans le tableau 5. La faible intensité de la réponse IGG1 est directement corrélée avec la présence de FL, qui inhibe la réponse à court terme (28 jours, J28) et à long terme (90 jours, J90).

De même, le tableau 6 présente les titres en IGG2.

Les résultats indiquent une corrélation entre le rapport P/N et la réponse IGG2 ; les formules 6 et 9 se démarquant des autres produits.

**Tableau 5 : Réponse anticorps de type IgG1 contre OVA dans la souris pour différentes huiles sous forme microémulsion H/E en fonction du temps.**

| **HUILE REF** | **FC (<C16)** | **RM** | **FL (>C28)** | **P/N** | **J14** | **J28** | **J42** | **J56** | **J90** |
|---|---|---|---|---|---|---|---|---|---|
| 2 | 4.9 | 21.5 | 7.1 | 1.9 | 12 800 | 16 000 | 96 000 | 48 000 | 48 000 |
| 3 | 4.2 | 20.5 | 1.5 | 1.9 | 9600 | 48 000 | 128 000 | 256 000 | 256 000 |
| 4 | 4.4 | 21.6 | 4.7 | 1.9 | 12 800 | 48 000 | 128 000 | 128 000 | 128 000 |
| 6 | 0.3 | 22.2 | 9.6 | 2.0 | 4 800 | 16 000 | 48 000 | 48 000 | 32000 |
| 9 | 2.0 | 19.3 | 2.2 | 2.8 | 6400 | 48 000 | 32 000 | 128 000 | 128 000 |
| 11 | 8.6 | 20.7 | 2.4 | 1.9 | 9600 | 48 000 | 128 000 | 256 000 | 256 000 |
| AlOH | | | | | 16 000 | 4 800 | 4 800 | 2400 | 2400 |

**Tableau 6 : Réponse anticorps de type IgG2 contre OVA dans la souris pour différentes huiles sous forme microémulsion H/E en fonction du temps.**

| **HUILE REF** | **FC (<C16)** | **RM** | **FL (>C28)** | **P/N** | **J14** | **J28** | **J42** | **J56** | **J90** |
|---|---|---|---|---|---|---|---|---|---|
| 2 | 4.9 | 21.5 | 7.1 | 1.86 | 400 | 12000 | 24 000 | 4000 | 400 |
| 3 | 4.2 | 20.5 | 1.5 | 1.86 | 600 | 16000 | 24 000 | 1 000 | 1000 |
| 4 | 4.4 | 21.6 | 4.7 | 1.86 | 150 | 8000 | 8000 | 1 000 | 1000 |
| 6 | 0.3 | 22.2 | 9.6 | 2.03 | 4800 | 16000 | 32000 | 8000 | 4000 |
| 9 | 2 | 19.3 | 2.2 | 2.85 | 1600 | 32000 | 48 000 | 96 000 | 96000 |
| 11 | 8.6 | 20.7 | 2.4 | 1.94 | 600 | 16000 | 24 000 | 1 000 | 4000 |
| AlOH | | | | | 150 | 150 | 150 | 300 | 300 |

Les comparaisons des ratios IgG1/IgG2 indiquent clairement une différence de comportement de la réponse pour les groupes vaccinés avec l'huile 9 et à moindre niveau avec l'huile 6 par rapport aux autres huiles (tableau 7).

**Tableau 7 : Ratio IgG1/IgG2 contre OVA dans la souris pour différentes huiles sous forme microémulsion H/E en fonction du temps.**

| **HUILE REF** | **FC (<C16)** | **RM** | **FL (>C28)** | **P/N** | **J14** | **J28** | **J42** | **J56** | **J90** |
|---|---|---|---|---|---|---|---|---|---|
| 2 | 4.9 | 21.5 | 7.1 | 1.86 | 32 | 1 | 4 | 12 | 120 |
| 3 | 4.2 | 20.5 | 1.5 | 1.86 | 16 | 3 | 5 | 256 | 256 |
| 4 | 4.4 | 21.6 | 4.7 | 1.86 | 85 | 6 | 16 | 128 | 128 |
| 6 | 0.3 | 22.2 | 9.6 | 2.03 | 1 | 1 | 2 | 6 | 8 |
| 9 | 2 | 19.3 | 2.2 | 2.85 | 4 | 2 | 1 | 1 | 1 |
| 11 | 8.6 | 20.7 | 2.4 | 1.94 | 16 | 3 | 5 | 256 | 64 |
| AlOH | | | | | 107 | 32 | 32 | 8 | 8 |

### Emulsion multiple Eau-dans-Huile-Eau (E/H/E) :

La formule adjuvante est cette fois composée d'un anhydromannitol octadecenoate éther de balance hydrophile lipophile (HLB) = 9, dispersé à 15% massique dans l'huile minérale à tester. La composition vaccinale est préparée en mélangeant à 30°C une part en poids d'adjuvant avec une part en poids de milieu antigénique (phase aqueuse) comprenant l'antigène OVA.

Essai sur souris : les résultats de l'essai souris pour les réponses IGG1 et pour les réponses IGG2 sont présentés respectivement dans les tableaux 8 et 9. En ce qui concerne la réponse cellulaire, l'évolution des valeurs des réponses IGG2 indique que l'huile 9 induit une réponse intense et durable, notamment après 28 jours.

Essai sur modèle bovin : dans ce cas, les mêmes vaccins ont été testés sur 5 bovins injectés avec 2 ml en sous-cutané ; les formules 9 et 11 ont été comparées. Les résultats sont présentés dans le tableau 10. Aucune différence n'est constatée pour les titres IGG1 (données non présentées). Un fort effet adjuvant est observé par rapport à l'antigène seul. La réponse IGG2 est très intense avec l'huile 9, faible avec l'huile 11 et inexistante pour l'antigène seul.

**Tableau 8 : Réponse anticorps de type IgG1 contre OVA dans la souris pour différentes huiles sous forme émulsion E/H/E en fonction du temps.**

| **Ref Huile** | **FC (<C16)** | **RM** | **FL (>C28)** | **P/N** | **J14** | **J28** | **J42** | **J56** | **J90** |
|---|---|---|---|---|---|---|---|---|---|
| 2.0 | 4.9 | 21.5 | 7.1 | 1.9 | 16000 | 32000 | 64000 | 64000 | 64000 |
| 3.0 | 4.2 | 20.5 | 1.5 | 1.9 | 16000 | 64000 | 128000 | 128000 | 128000 |
| 4.0 | 4.4 | 21.6 | 4.7 | 1.9 | 16000 | 64000 | 128000 | 128000 | 128000 |
| 6.0 | 0.3 | 22.2 | 9.6 | 2.0 | 16 000 | 64000 | 64 000 | 64 000 | 64000 |
| 9.0 | 2.0 | 19.3 | 2.2 | 2.8 | 16 000 | 64000 | 128000 | 128000 | 128000 |
| 11.0 | 8.6 | 20.7 | 2.4 | 1.9 | 16 000 | 64000 | 128000 | 128000 | 128000 |
| AlOH | | | | | 16 000 | 4 800 | 4 800 | 2400 | 2400 |

**Tableau 9 : Réponse anticorps de type IgG2 contre OVA dans la souris pour différentes huiles sous forme émulsion E/H/E en fonction du temps.**

| **Ref Huile** | **FC (<C16)** | **RM** | **FL (>C28)** | **P/N** | **J14** | **J28** | **J42** | **J56** | **J90** |
|---|---|---|---|---|---|---|---|---|---|
| 2 | 4.9 | 21.5 | 7.1 | 1.86 | 400 | 12 000 | 24 000 | 4000 | 400 |
| 3 | 4.2 | 20.5 | 1.5 | 1.86 | 400 | 12 000 | 24 000 | 4000 | 400 |
| 4 | 4.4 | 21.6 | 4.7 | 1.86 | 400 | 12 000 | 24 000 | 4000 | 400 |
| 6 | 0.3 | 22.2 | 9.6 | 2.03 | 400 | 4000 | 12 000 | 4000 | 400 |
| 9 | 2 | 19.3 | 2.2 | 2.85 | 9600 | 16 000 | 128 000 | 25600 0 | 256000 |
| 11 | 8.6 | 20.7 | 2.4 | 1.94 | 300 | 600 | 1 200 | 1200 | 1200 |
| AlOH | | | | | 150 | 150 | 150 | 300 | 300 |

**Tableau 10 : Réponse anticorps de type IgG2A contre OVA dans le bovin pour les huiles 9 et 11, sous forme émulsion E/H/E en fonction du temps.**

| **Ref Huile** | **FC (<C16)** | **RM** | **FL (>C28)** | **P/N** | **J0** | **J28** | **J60** | **J140** |
|---|---|---|---|---|---|---|---|---|
| 9 | 2 | 19.3 | 2.2 | 2.85 | - | 600 | 6000 | 10 000 |
| 11 | 8.6 | 20.7 | 2.4 | 1.94 | - | 10 | 600 | 2000 |

Par améliorer la composante cellulaire de la réponse immunitaire, on entend :
- A) obtenir une réponse immunitaire se caractérisant par un ratio IgG1/IgG2 compris entre 0,25 et 4, et
- B) obtenir une réponse immunitaire dont la composante cellulaire est aussi intense, à savoir caractérisée par un IgG2 supérieur ou égal à 32 000 après le rappel de vaccination pratiqué après 28 jours et après la mesure du titre IGG2 à cette date (réponse secondaire et long terme).

### A- ratio IgG1/IgG2

### a) cas des adjuvants vaccins se présentant sous la forme d'une émulsion E/H/E, les résultats expérimentaux IgG1/IgG2 sont les suivants :

- pour l'huile 6 : 40 (J14), 16 (J28), 5,3 (J42), 16 (J56) et 160 (J90).
- pour l'huile 11 : 53.3 (J14), 107 (J28), 107 (J42), 107 (J56) et 107 (J90).
- pour l'huile 9 : 1.7 (J14), 4 (J28), 1 (J42), 0.5 (J56) et 0.5 (J90).
L'huile 9 permet d'obtenir un ratio IgG1/IgG2 compris entre 0,25 et 4 avant et après le rappel de vaccination à J28 (le rappel de vaccination réalisé à J28, est effectué après la mesure de la réponse immunitaire).

### b) cas des adjuvants vaccins se présentant sous la forme d'une microémulsion H/E, la déclaration d'invention comprend les résultats expérimentaux IgG1/IgG2 suivant :

▪ pour l'huile 6 : 1 (J14), 1 (J28), 2 (J42), 6 (J56) et 8 (J90)
▪ pour l'huile 11 : 16 (J14), 3 (J28), 5 (J42), 256 (J56) et 64 (J90)
▪ pour l'huile 9 : 4 (J14), 2 (J28), 1 (J42), 1 (J56) et 1 (J90)

Pour l'huile 9, le ratio IgG1/IgG2 compris entre 0,25 et 4 est toujours obtenu avant le rappel de vaccination à J28, et reste constant dans le temps.

Pour l'huile 6, le ratio IgG1/IgG2 compris entre 0,25 et 4 est aussi obtenu avant le rappel de vaccination (réponse primaire) mais ne subsiste pas dans le temps lors de la mesure de la réponse secondaire (après le rappel de vaccination de J28) et de la réponse à long terme (J90).

Pour l'huile 11, le ratio IgG1/IgG2 compris entre 0,25 et 4 n'est pas observé pour la réponse secondaire ni pour la réponse à long terme.

### c) cas des adjuvants vaccins se présentant sous la forme d'une émulsion E/H, la déclaration d'invention comprend les résultats expérimentaux IgG1/IgG2 suivant :

▪ pour l'huile 9 : 1 (J14), 1 (J28), 1 (J42), 1 (J56) et 1 (J90) Tous ces résultats enregistrés montrent de bons résultats pour le ratio IgG1/IgG2.

### B- Intensité des réponses IgG2.

### a) cas des adjuvants vaccins se présentant sous la forme d'une émulsion E/H/E, les résultats expérimentaux IgG2 sont les suivants (tableau 9):

a. pour l'huile 6 (J14), 4 000 (J28), 12 000 (J42), 4 000 (J56) et 400 (J90)
b. pour l'huile 11: 300 (J14), 600 (J28), 1200 (J42), 1200 (J56) et 1200 (J90)
c. pour l'huile 9 : 9600 (J14), 16 000 (J28), 128 000 (J42), 256 000 (J56) et 256 000 (J90)
L'huile 9 permet d'avoir une réponse cellulaire intense (selon les critères définis précédemment) après le rappel de vaccination pratiqué après 28 jours.

### b) cas des adjuvants vaccins se présentant sous la forme d'une microémulsion H/E, la déclaration d'invention comprend les résultats expérimentaux IgG2 suivant (tableau 6):

a. pour l'huile 6 : 4800 (J14), 16 000 (J28), 32 000 (J42), 8 000 (J56) et 4000 (J90)
b. pour l'huile 11: 600 (J14), 16 000 (J28), 24 000 (J42), 1000 (J56) et 40000 (J90)
c. pour l'huile 9: 1600 (J14), 32 000 (J28), 48 000 (J42), 96 000 (J56) et 96 000 (J90)
L'huile 9 permet d'avoir une réponse cellulaire intense (selon les critères définis précédemment) pour la réponse secondaire (après le rappel de vaccination) et pour la réponse à long terme (J90).

### c) cas des adjuvants vaccins se présentant sous la forme d'une émulsion E/H, la déclaration d'invention comprend les résultats expérimentaux IgG2 suivant (tableau 4):

a. *pour* l'huile 9: 9600 (J14), 16 000 (J28), 128 000 (J42), 256 000 (J56) et 256 000 (J90)

Tous ces résultats enregistrés montrent de bons résultats pour la réponse IgG2.

D'après ces résultats il est bien montré qu'une composition d'une huile minérale destinée à être utilisée comme adjuvant de vaccin injectable, objet de la présente invention, comme l'huile 9 par exemple, présentant les caractéristiques suivantes :
- FC < 10% et FL < 5% ;
- P/N compris entre 2,5 et 3 (2,85 environ ici) ;
garantit une bonne innocuité, une bonne efficacité quant à la réponse humorale et une forte réponse cellulaire à la fois sous forme d'émulsion E/H et E/H/E et microémulsion H/E.

### II) DEUXIEME PARTIE

### Série d'ESSAIS 1 : travail expérimental sur un modèle « poulet »

Dans le cadre des essais expérimentaux mis en oeuvre, l'efficacité de la réponse pour chaque formule repose sur les critères suivants :
a) titre d'anticorps dans les sérums ;
b) infiltration de cellules blanches au site d'injection ;
c) épreuve de virulence consistant à inoculer aux animaux 10 000 oocystesde la souche Emeria Acervulina deux semaines après le traitement, et comparaison :
   a. de la prise de poids des animaux
   b. du nombre de parasites excrétés
Le Tableau 11 indique les différentes formules qui ont été testées :

**Tableau 11**

| GROUPE | A | B | C | D | E | F |
|---|---|---|---|---|---|---|
| EMULSION | E/H | E/H | E/H/E | E/H/E | CFA | Sans émulsion |
| HUILE | 1 | 2 | 1 | 2 | Minérale standard | Sans huile |

Les huiles impliquées dans les essais expérimentaux sont caractérisées dans le tableau ci-dessous

| **Ref Huile** | **FC (<C16)** | **RM** | **FL (>C28)** | **P/N** | **RL** |
|---|---|---|---|---|---|
| Minérale Standard | 4.9 | 21.5 | 7.1 | 1.86 | 0.5 |
| Huile 1 (H1) | 2 | 19.3 | 2.2 | 2.85 | 0 |
| Huile 2 (H2) | 8.6 | 20.7 | 2.4 | 1.94 | 0.5 |

Les formulations des groupes A et B, à savoir les émulsions E/H1 (Groupe A) et les émulsions E/H2 (Groupe B), comprennent pour 100% de leur masse :
- 30 % massique d'une phase aqueuse comprenant l'antigène et de l'eau
- 70% massique d'adjuvant huileux comprenant pour 100% de sa masse :
   ∘ 15 % massique d'oléate de mannitan
   ∘ 85% massique d'huile (Huile 1 ou Huile 2)

L'antigène, la protéine 3-1 E, est présent dans ces formulations en une proportion telle qu'une dose de formulation injectée comprend 50 µg d'antigène, le volume de la dose de formulation injectée étant de 100 µL.

Les formulations des groupes C et D, à savoir les micro-émulsions E/H1/E (Groupe C) et les micro-émulsions E/H2/E (Groupe D), comprennent pour 100% de leur masse :
- 90% massique d'une phase aqueuse comprenant l'antigène et de l'eau
- 10% massique d'adjuvant huileux comprenant pour 100% de sa masse :
   ∘ 40 % massique d'oléate de sorbitan éthoxylé à 20 moles d'oxyde d'éthylène Polysorbate 80)
   ∘ 60 % massique d'huile (Huile 1 ou Huile 2).

L'antigène, la protéine 3-1 E, (sont) présent(s) dans ces formulations en une proportion telle qu'une dose de formulation injectée comprend 50 µg d'antigène, le volume de la dose de formulation injectée étant de 100 µL.

Au niveau des titres d'anticorps dans les sérums tous les groupes testés ont présenté des titres anticorps sériques importants et équivalents sauf le groupe comportant la protéine seule (Groupe F). La réponse humorale obtenu pour chacun des groupes est à un niveau élevé et satisfaisant (résultats non présentés).

Au niveau des infiltrations de cellules blanches au site d'injection, les intensités des infiltrations locales desdites cellules blanches (lymphocytes et macrophages) ont été évaluées par immunofluorescence indirecte sur des coupes de tissus pour les différents groupes.

Des biopsies ont été pratiquées sur 3 animaux de chaque groupe, au niveau du site d'injection, de façon à obtenir des échantillons de la peau de ces animaux. Cette biopsie a été réalisée un jour après la seconde immunisation desdits animaux, soit 28 jours après la première immunisation. Ces immunisations ont consisté en des injections d'un mélange de profiline et de formulations testées. Un témoin, consistant en une injection de profiline sans milieu vaccinal, a également été réalisé dans les mêmes conditions opératoires. Les échantillons ainsi obtenus ont été immédiatement congelés dans un milieu contenant de l'azote liquide et conservés à une température de - 20°C. Des morceaux de 5 µm de chaque échantillon ont été ensuite installés sur des supports préalablement nettoyés, et lavés dans l'acétone pendant 20 minutes à une température de 4°C, et « fixés » par un sérum de cheval à 10% pendant 20 minutes à une température de 20°C. Des anticorps CD8 du poulet, dilués au 1/200^{ème}, ont été ajoutés, puis le mélange en résultant a été incubé à une température ambiante (20°C) pendant 2 heures. Les supports ont ensuite été lavés avec une solution tampon de phosphate et incubés avec un anticorps secondaire IgG du poulet « Alexa Fluor 488-labeled antichicken » (commercialisé sous l'appellation Invitrogen par la société Carlsbad) à une dilution de 1/500ème pendant 2 heures à température ambiante (20°C). Les supports ont été ensuite mis en contact avec un révélateur colorimétrique, le Fluoromount-G, et observés avec un microscope électronique (commercialisé sous l'appellation LSM 510 META par la société Carl Zeiss).

Par ce protocole, la visualisation des cellules CD8+ a été appréciée par une notation selon l'échelle suivante :
- notation « 0 » : absence de tâches de cellules CD8+ observées,
- notation « +» : observation d'une présence, d'une faible densité, de cellules CD8+ ,
- notation « ++ » : observation d'une forte présence, d'une densité élevée, de cellules CD8+, et
- notation « +++ » : observation d'une très forte présence, d'une densité très élevée, de cellules CD8+.

Plus la quantité et la densité de lymphocytes et de macrophages observées au niveau du site d'injection est importante, plus la capacité à développer une réponse immunitaire efficace sera possible pour le vaccin testé.
Les résultats obtenus sont indiqués dans le tableau 12

**TABLEAU 12**

| **GROUPE** | **A** | **B** | **C** | **D** | **E** | **F** |
|---|---|---|---|---|---|---|
| Adjuvant | E/H1 | E/H2 | E/H1/E | E/H2/E | CFA | SANS ADJUVANT |
| Antigène | Oui | Oui | Oui | Oui | Oui | Oui |
| Lymphocyte | +++ | + | ++ | ++ | + | 0 |
| Macrophage | ++ | + | + | + | + | 0 |

Par rapport au vaccin comprenant l'adjuvant de l'état de la technique (Groupe E), les vaccins selon l'invention améliorent la production de lymphocytes sur le site d'injection pour les groupes D, C et A; alors que pour les macrophages, seul le vaccin selon l'invention du groupe A permet d'améliorer la production de macrophages et les autres vaccins selon l'invention permettent d'obtenir des résultats de production de macrophages sur le site d'injection similaire au vaccin comprenant l'adjuvant de l'état de la technique.

Au niveau de l'épreuve de virulence, une protection efficace par le vaccinimpliquera que les animaux :
- présenteront une prise de poids similaire par rapport aux témoins non vaccinés et non éprouvés ;
- présenteront une réduction de la charge parasitaire (évaluée par le nombre de parasites excrétés) par rapport aux animaux éprouvés et non vaccinés.

De façon générale, l'épreuve virulente correspond à l'administration d'une dose précise et maîtrisée de l'agent pathogène contre lequel les animaux ont été préalablement vaccinés. La protection induite par les vaccins expérimentaux est évaluée selon divers critères en fonction du modèle animal et de la nature du pathogène.

Deux critères principaux ont étés suivis post épreuve virulente :
- Prise de poids des animaux 10 jours post infection, et
- Réduction du nombre de pathogènes excrétés.

### o Prise de poids des animaux 10 iours post infection.

Le tableau 13 résume les résultats obtenus lors de vaccination et d'infection de poulet contre la coccidiose aviaire (souche utilisée *Emeria acervulina).* Le pouvoir pathogène de cette souche est important et se traduit par une modification de la prise de poids des animaux infectés.

**TABLEAU 13 : prise des poids des différents groupes après épreuve virulente.**

| **GROUPE** | **A** | **B** | **C** | **D** | **E** | **F** | **G** | **H** |
|---|---|---|---|---|---|---|---|---|
| EMULSION | E/H1 | E/H2 | E/H1/E | E/H2/E | CFA | SANS ADJUVANT | SANS VACCIN | SANS VACCIN |
| Antigène | Oui | Oui | Oui | Oui | Oui | Oui | Non | Non |
| EPREUVE VIRULENTE | 10 000 oocystes Emeria acervulina | | | | | | | SANS EPREUVE |
| PRISE DE POIDS DIX JOURS APRES EPREUVE (g) | 834 | 798 | 802 | 740 | 773 | 735 | 730 | 850 |

On peut observer que pour des sujets éprouvés, la vaccination sans adjuvant n'induit pas de protection en termes de prise de poids : les témoins sans adjuvant (Groupe F) et sans vaccin (Groupe G) induisant des résultats similaires.

Par rapport au sujet éprouvé et non vacciné (groupe G), les vaccins selon l'invention comprenant un adjuvant à base d'huiles H1 et H2 permettent des prises de poids significatives à l'exception des vaccins du Groupe D.

Par rapport au vaccin comprenant l'adjuvant de l'état de la technique (Groupe E), les vaccins selon l'invention induisent une prise de poids supérieure à l'exception du vaccin se présentant sous la forme d'une émulsion E/H2/E (Groupe D).

Il faut également remarquer que les vaccins selon l'invention comprenant un adjuvant à base d'huile 1 induisent une prise de poids plus importante que les vaccins selon l'invention comprenant un adjuvant à base d'huile 2.

Pour les vaccins selon l'invention du groupe A, la prise de poids n'est inférieure que de 1,9% par rapport à la prise de poids des sujets non éprouvés.

Par ailleurs, pour chacune des huiles minérales considérées (H1 ou H2), on remarquera que les vaccins expérimentaux formulés en émulsion eau/huile semblent plus protecteurs que les formules eau/huile/eau :
- pour l'huile 1, la prise de poids pour un vaccin sous la forme d'une émulsion E/H1 (groupe A) est de 834 g, soit une prise de poids de 99g par rapport aux animaux du groupe sans adjuvant (groupe F), alors que pour un vaccin sous la forme d'une émulsion E/H1/E la prise de poids (groupe C) est de 802 grammes et présente une prise de poids de 67g par rapport aux animaux du groupe sans adjuvant (groupe F).
- pour l'huile 2, la prise de poids pour un vaccin sous la forme d'une émulsion E/H2 (groupe B) est de 798 g, soit une prise de poids de 63g par rapport aux animaux du groupe sans adjuvant (groupe F) alors que pour un vaccin sous la forme d'une émulsion E/H2/E la prise de poids (groupe D) est de 740 grammes, et présente une prise de poids de 5g par rapport aux animaux du groupe sans adjuvant (groupe F).

### o Réduction du nombre de pathogène excrété :

Les résultats obtenus sont compris dans le tableau 14.
Les résultats sont exprimés en réduction du nombre d'oocystes excrétés (plus les animaux sont infectés, plus ils excrètent, donc moins ils étaient protégés).

**TABLEAU 14**

| **GROUPE** | **A** | **B** | **C** | **D** | **E** | **F** |
|---|---|---|---|---|---|---|
| Adjuvant | E/H1 | E/H2 | E/H1/E | E/H2/E | CFA | SANS ADJUVANT |
| Antigène | Oui | Oui | Oui | Oui | Oui | Oui |
| Moyenne excrétée (X10E8) | 1.42 | 2.75 | 2.54 | 3.09 | 2.13 | 2.86 |
| ECART TYPE | 0.23 | 0.52 | 0.8 | 0.39 | 0.49 | 0.36 |

Par rapport au témoin du groupe F (antigène mais sans adjuvant), les vaccins selon l'invention induisent donc une diminution de la moyenne excrétée.

Par rapport au Groupe E (Antigène + adjuvant de l'état de la technique), le vaccin selon l'invention du groupe 1 procure une diminution de la moyenne excrétée.

De plus, l'écart type pour le Groupe A est le plus faible, indiquant une réponse plus homogène des animaux vaccinés. Ce critère est un point important car cela signifie que les susceptibilités individuelles de réponse biologique des animaux sont gommées par l'efficacité du vaccin.

### CONCLUSION ESSAI 1

Les vaccins comprenant les adjuvants selon l'invention permettent d'apporter une réponse immunitaire améliorée et une meilleure efficacité pour prévenir les volailles de la coccidiose.

D'autre part, le vaccin selon l'invention se présentant sous la forme d'une émulsion E/H1, avec H1 caractérisé par un ratio P/N>2 présente comme avantage de stimuler une plus grande production de cellules blanches au niveau du site d'injection, et de concilier, suite à une épreuve de virulence avec la souche Emeria Acervulina, à la fois la prise de poids la plus élevée et une réduction très significative de l'excrétion d'oocystes.
**Série d'ESSAIS 2 :** Compte tenu de la série d'ESSAIS 1 et de ses conclusions, à savoir l'observation de meilleures performances pour un vaccin comprenant :
- La protéine recombinante comportant la séquence 3-1 *d'Eimera Acervulina.*
- L'huile 1
- De l'eau
- Un système tensioactif
et se présentant sous la forme d'une émulsion E/H , les inventeurs ont réalisés deux types d'épreuves de virulence :
- une épreuve de virulence avec la souche Emeria Acervulina (AE),
- épreuve de virulence avec la souche Emeria Tenella (ET).
Les formulations testées, à savoir les émulsions E/H1, comprennent pour 100% de leur masse :
- 30% massique d'une phase aqueuse comprenant l'antigène et de l'eau
- 70% massique d'adjuvant huileux comprenant pour 100% de sa masse :
   ∘ 15% massique d'oléate de mannitan ;
   ∘ 85% massique d'huile (Huile 1 ou Huile 2).

L'antigène, la protéine 3-1 E, est présent dans ces formulations en une proportion telle qu'une dose de formulation injectée comprend 30 µg d'antigène, le volume de la dose de formulation injectée étant de 100 µL.

Pour identifier des protections croisées élargissant les propriétés du vaccin expérimental testé. En effet, la réponse immunitaire induite de type cellulaire, permet alors de protéger contre différentes souches de parasites.

La dose de protéine utilisée est de 30 µg / ml. L'épreuve virulente est effectuée par administration de 10 000 oocystes par oiseau (tableau 15).

Les animaux sont ensuite suivis sur les paramètres de prise de poids, d'anticorps, de prolifération des lymphocytes de rate après restimulation antigénique ou par mitogènes (pour quantifier la réponse cellulaire) et protection lors d'une épreuve virulente.
*E. acervulina :* elle est modérément pathogène.
   Les lésions se localisent dans l'intestin grêle surtout au duodénum, avec des tâches puis des stries blanchâtres dans la muqueuse = lésions « en échelle ». Les lésions sont causées par les oocystes.
*E. tenella :* c'est la coccidie la plus pathogène, les lésions étant causées par les schizontes.

Les lésions sont localisées dans les caeca, qui sont remplis de sang, peuvent se rompre ou être gangréneux. La carcasse peut être anémiée. La mortalité est souvent élevée.
Le tableau 15 décrit les différents groupes testés :

**TABLEAU 15**

| **GROUPE** | **A** | **B** | **C** | **D** | **E** | **F** | **G** | **H** | **I** |
|---|---|---|---|---|---|---|---|---|---|
| Description | Non vacciné | Non vacciné | Non vacciné | vacciné | vacciné | vacciné | vacciné | vacciné | vacciné |
| Antigène 3-1 E (30 µg / ml) | Non | Non | Non | Oui | Oui | Oui | Oui | Oui | Oui |
| Adjuvant | Non vacciné | Non vacciné | Non vacciné | Non | Non | CFA | CFA | E/H1 | E/H1 |
| Souche Epreuve Virulence | Non éprouvé | EA | ET | EA | ET | EA | ET | EA | ET |

| | | | | | | | | | |
|---|---|---|---|---|---|---|---|---|---|
| CFA : Complete Freund Adjuvant (adjuvant de l'état de la technique) | | | | | | | | | |

Les poulets vaccinés dans les différents groupes sont ensuite suivis selon les différents critères suivants :
a) prise de poids;
b) production de titres anticorps (sérologie) ;
c) évaluation des intensités des infiltrations locales des cellules blanches (lymphocytes) par la méthode d'immunofluorescence indirecte sur des prélèvements de splénocytes de rate réalisés 48 h après la vaccination (ou immunisation). Ces immunisations ont consisté en des injections d'un mélange de profiline et de formulations testées. Un témoin, consistant en une injection de profiline sans milieu vaccinal, a également été réalisé dans les mêmes conditions opératoires. Le mode opératoire utilisé est identique à celui mis en oeuvre sur les prélèvements de peaux des animaux décrit ci-dessus, à savoir :
   - conservation des échantillons prélevés de splénocytes à -20°C
   - installation des prélèvements des splénocytes sur des supports et lavage dans l'acétone pendant 20 minutes à 20°C
   - ajout d'anticorps CD8 dilués au 1/200^{ème} et incubation subséquente pendant 2 heures à 20°C
   - lavage des supports obtenus avec une solution tampon de phosphate, et incubation subséquente avec un anticorps secondaire IgG du poulet « Alexa Fluor 488-labeled antichicken » à une dilution de 1/500^{ème} pendant 2 heures à température ambiante (20°C)
   - mise en contact avec le révélateur colorimétrique Fluoromount-G
   - observation avec un microscope électronique (commercialisé sous l'appellation LSM 510 META par la société Carl Zeiss).
   Par à ce protocole, la visualisation des cellules CD8+ (lymphocytes) a été appréciée par une notation selon l'échelle suivante :
   - notation « 0 » : absence de tâches de cellules CD8+ observées
   - notation « +» : observation d'une présence, d'une faible densité, de cellules CD8+
   - notation « ++ » : observation d'une forte présence, d'une densité élevée, de cellules CD8+
   - notation « +++ » : observation d'une très forte présence, d'une densité très élevée, de cellules CD8+ ;
d) nombre d'oocystes excrétés.

### Résultats:

- prises de poids : les groupes vaccinés par le vaccin se présentant sous la forme d'une émulsion E/H1 se caractérisent par une prise de poids similaire à celle observée par les autres groupes (résultats non présentés) ;
- sérologie : production élevée de titres anticorps, supérieure à celle observée pour les groupes vaccinés avec le vaccin présentant comme adjuvant le CFA et à celle observée pour les groupes vaccinés avec un vaccin ne comprenant pas d'adjuvant (résultats non présentés) ;
- prolifération des lymphocytes : les notations les plus élevées sont obtenues pour les groupes H et I vaccinés avec le vaccin se présentant sous la forme E/H et comprenant l'adjuvant avec l'huile H1.

| GROUPE | A | B | C | D | E | F | G | H | I |
|---|---|---|---|---|---|---|---|---|---|
| Index Prolifération lymphocytaire | 0 | 0 | 0 | 0 | 0 | 0 | 0 | ++ | ++ |

- nombre d'oocystes excrétés:
Les tableaux 16 et 17 indiquent les résultats du nombre d'oocystes excrétés pour respectivement les groupes ayant subi l'épreuve de virulence avec *Emeria Acervulina et Emeria Tenella.*

**Tableau 16 : Résultats d'excrétion après épreuve virulente par la souche EA**

| GROUPE | B | D | F | H |
|---|---|---|---|---|
| Moyenne (X 10 E 8) | 3.44 | 3.33 | 3.30 | 2.71 |
| Ecart type | 0.50 | 0.67 | 0.89 | 0.27 |

**Tableau 17 : Résultats d'excrétion après épreuve virulente par la souche ET.**

| GROUPE | C | E | G | I |
|---|---|---|---|---|
| Moyenne (X 10 E 8) | 1.49 | 1.20 | 1.11 | 0.95 |
| Ecart type | 0.23 | 0.27 | 0.42 | 0.13 |

Pour les deux souches, les groupes vaccinés avec le vaccin comprenant l'adjuvant à base de H1 montrent une moyenne d'excrétion inférieure à celle observée sur les groupes vaccinés avec le vaccin comprenant l'adjuvant de l'état de la technique (CFA). Il en est de même de la comparaison avec les groupes vaccinés avec un vaccin ne contenant pas d'adjuvant (groupes D et E).

## Revendications

1. Adjuvant de vaccin comprenant pour 100% de sa masse :
- de 10 % à 95 % d'une huile minérale comprenant :
- de 0,05% massique à 10% massique de chaînes hydrocarbonées ayant moins de 16 atomes de carbone ;
- de 0,05% massique à 5% massique de chaînes hydrocarbonées ayant plus de 28 atomes de carbone ;
- et possédant un rapport P/N, correspondant au rapport de la quantité massique des chaînes hydrocarbonées de type paraffinique sur la quantité massique des chaînes hydrocarbonées de type naphténique, compris entre 2,5 et 3.

2. Utilisation d'un adjuvant de vaccin tel que défini à la revendication 1 pour la fabrication d'une composition vaccinale destinée à la prévention contre la coccidiose.

3. Utilisation selon la revendication 2, pour la fabrication d'une composition vaccinale destinée à la prévention contre la coccidiose chez l'animal, en particulier les volailles.

4. Vaccin pour utilisation dans la prévention contre la coccidiose comprenant l'adjuvant tel que défini la revendication 1 ainsi qu'au moins un antigène.

5. Vaccin pour utilisation selon la revendication 4, pour utilisation dans la prévention contre la coccidiose chez l'animal, en particulier les volailles.

6. Vaccin pour utilisation selon l'une des revendications 4 ou 5 **caractérisé en ce que** les coccidies sont choisies dans le groupe constitué par *Eimeria, Isospora, Toxoplasma, Besnoitia, Neospora* de préférence *Eimera.*

7. Vaccin pour utilisation selon la revendication 6 **caractérisé en ce qu'**il est sous forme d'émulsion du type eau dans huile (E/H).

8. Vaccin pour utilisation selon l'une des revendications 4 à 7 **caractérisé en ce que** l'antigène est une protéine recombinante comportant la séquence 3-1 *d'Eimera Acervulina.*

9. Utilisation d'une huile minérale pour la fabrication d'un vaccin tel que défini à l'une des revendications 4 à 8, **caractérisée en ce que** la dite huile comprend pour 100% de sa masse :
- de 0,05% à 10% de chaînes hydrocarbonées ayant moins de 16 atomes de carbone ;
- de 0,05% à 5% de chaînes hydrocarbonées ayant plus de 28 atomes de carbone ;
et possède un rapport P/N, correspondant au rapport de la quantité massique de chaînes carbonées de type paraffinique sur la quantité massique de chaînes carbonées de type naphténique, compris entre 2,5 et 3.

## Patentansprüche

1. Impfstoffadjuvans, umfassend für 100 % seiner Masse:
- von 10 % bis 95 % eines Mineralöls, umfassend:
-- von 0,05 Masseprozent bis 10 Massenprozent Kohlenwasserstoffketten mit mindestens 16 Kohlenstoffatomen;
-- von 0,05 Masseprozent bis 5 Massenprozent Kohlenwasserstoffketten mit mehr als 28 Kohlenstoffatomen;
-- und aufweisend eine Beziehung P/N, die der Beziehung zwischen der Massenmenge der Kohlenwasserstoffketten der paraffinischen Art zur Massenmenge der Kohlenwasserstoffketten der naphtenischen Art entspricht, die zwischen 2,5 und 3 liegt.

2. Verwendung eines Impfstoffadjuvans, wie in Anspruch 1 definiert, zur Herstellung einer Impfzusammensetzung, die für die Prävention gegen Kokzidiose ausgelegt ist.

3. Verwendung nach Anspruch 2 zur Herstellung einer Impfzusammensetzung, die für die Prävention gegen Kokzidiose bei Tieren, insbesondere Geflügel, ausgelegt ist.

4. Impfstoff zur Verwendung bei der Prävention der Kokzidiose, umfassend das Adjuvans, wie in Anspruch 1 definiert, ebenso wie mindestens ein Antigen.

5. Impfstoff zur Verwendung nach Anspruch 4 zur Verwendung bei der Prävention der Kokzidiose, bei Tieren, insbesondere Geflügel.

6. Impfstoff zur Verwendung nach einem der Ansprüche 4 oder 5, **dadurch gekennzeichnet, dass** die Kokzidiosen ausgewählt sind aus der Gruppe, bestehend aus *Eimeria, Isospora, Toxoplasma, Besnoitia, Neospora* vorzugsweise *Eimera.*

7. Impfstoff zur Verwendung nach Anspruch 6, **dadurch gekennzeichnet, dass** er die Form einer Emulsion der Art Wasser-in-Öl (E/H) umfasst.

8. Impfstoff zur Verwendung nach einem der Ansprüche 4 bis 7, **dadurch gekennzeichnet, dass** das Antigen ein rekombinantes Protein ist, umfassend die Sequenz 3-1 von *Eimera Acervulina.*

9. Verwendung eines Mineralöls zur Herstellung eines Impfstoffs, wie in einem der Ansprüche 4 bis 8 definiert, **dadurch gekennzeichnet, dass** das Öl für 100 % seiner Masse Folgendes umfasst:
- 0,05 % bis 10 % Kohlenwasserstoffketten mit mindestens 16 Kohlenstoffatomen;
- 0,05 %bis 5 % Kohlenwasserstoffketten mit mehr als 28 Kohlenstoffatomen;
- und eine Beziehung P/N aufweist, die der Beziehung zwischen der Massenmenge der Kohlenwasserstoffketten der paraffinischen Art zur Massenmenge der Kohlenwasserstoffketten der naphtenischen Art entspricht, die zwischen 2,5 und 3 liegt.

## Claims

1. Vaccine adjuvant comprising, for 100% of its mass:
- from 10% to 95% of a mineral oil comprising:
-- from 0.05% by mass to 10% by mass hydrocarbon chains having fewer than 16 carbon atoms;
-- 0.05% by mass to 5% by mass hydrocarbon chains having more than 28 carbon atoms;
-- and having a P/N ratio, corresponding to the ratio of the quantity by mass of hydrocarbon chains of the paraffin type to the quantity by mass of hydrocarbon chains of the naphthene type, of between 2.5 and 3.

2. Use of a vaccine adjuvant as defined in claim 1 for manufacturing a vaccine composition intended for preventing coccidiosis.

3. Use according to claim 2, for manufacturing a vaccine composition intended for preventing coccidiosis in animals, in particular poultry.

4. Vaccine for use in the prevention of coccidiosis, comprising the adjuvant as defined in claim 1 as well as at least one antigen.

5. Vaccine for use according to claim 4, for use in the prevention of coccidiosis in animals, in particular poultry.

6. Vaccine for use according to one of claims 4 or 5, **characterised in that** the coccidia are chosen from the group consisting of *Eimeria, Isospora, Toxoplasma, Bensnoitia, Neospora* and preferably *Eimera.*

7. Vaccine for use according to claim 6, **characterised in that** it is in the form of an emulsion of the water in oil (W/O) type.

8. Vaccine for use according to one of claims 4 to 7, **characterised in that** the antigen is a recombinant protein comprising the sequence 3-1 of *Eimera Acervulina.*

9. Use of a mineral oil for manufacturing a vaccine as defined in any of claims 4 to 8, **characterised in that** said oil comprises, for 100% of its mass:
-- from 0.05% to 10% hydrocarbon chains having fewer than 16 carbon atoms;
-- 0.05% to 5% hydrocarbon chains having more than 28 carbon atoms;
and has a P/N ratio, corresponding to the ratio of the quantity by mass of hydrocarbon chains of the paraffin type to the quantity by mass of hydrocarbon chains of the naphthene type, of between 2.5 and 3.
